# EUROPEAN PATENT APPLICATION

(11) **EP 4 349 190 A1**
(43) Date of publication of application: **10.04.2024**
(21) Application number: 22811692.7
(22) Date of filing: 27.05.2022
(51) Int. Cl.: A24F 40/40, A24F 40/42, A24F 40/50, A24F 40/85, A61L 2/08, H01F 7/00

(54) **ELECTRONIC CIGARETTE**

(30) Priority: 28.05.2021 KR 20210069246
(71) Applicant: Shin, Jong-Soo, Cheongju-si, Chungbuk 28582 (KR)
(72) Inventor: Shin, Jong-Soo, Cheongju-si, Chungbuk 28582 (KR)
(74) Representative: Witte, Weller & Partner Patentanwälte mbB
(86) International application number: PCT/KR2022/007588
(87) International publication number: WO 2022/250501

(57) **Abstract**

The present invention relates to an electronic cigarette and, more particularly, an electronic cigarette in which, in a state where a cover part is separated from a body part, thereby exposing a cartridge part to the outside, a current flows toward the cartridge part when a user presses a button formed on the electronic cigarette, and thus gas may be generated, but, in a state where the cover part is coupled to the body part, thereby covering the cartridge part, the current is prevented from flowing toward the cartridge part even when the button formed on the electronic cigarette is pressed, and thus the problem where the gas is generated as a result of the current being supplied toward the cartridge part by the button being pressed against the intention of the user is prevented, electrical discharge of the electronic cigarette as a result of unnecessary power consumption is prevented, and the cartridge part may be sterilized for a predetermined time from the time point when the cover part is coupled to the body part.

## Description

### Technical Field

The present disclosure relates to an electronic cigarette and, more particularly, to an electronic cigarette in which, in a state where a cover part is separated from a body part, thereby exposing a cartridge part to the outside, a current flows toward the cartridge part when a user presses a button formed on the electronic cigarette, and thus aerosol may be generated, but, in a state where the cover part is coupled to the body part, thereby covering the cartridge part, the current is prevented from flowing toward the cartridge part even when the button formed on the electronic cigarette is pressed, and thus the problem where the aerosol is generated as a result of the current being supplied toward the cartridge part by the button being pressed against the intention of the user is prevented, electrical discharge of the electronic cigarette as a result of unnecessary power consumption is prevented, and the cartridge part may be sterilized for a predetermined time from the time point when the cover part is coupled to the body part.

### Background Art

Tobacco smoke from cigarettes, cigars, pipe tobacco, etc., contains a variety of substances harmful to the human body, such as tar, hydrocarbons, and carbon monoxide, which cause various diseases such as lung cancer and circulatory diseases. While mainstream (firsthand) smoke that a smoker inhales is obviously harmful to smoker's health, secondhand smoke, the combination of the smoke that a smoker exhales and toxic compounds in smoke lingering in spaces where smoking takes place, has adverse effects on people around a smoker.

Unlike in the past, policies are being implemented around the world to reduce smoking rates and promote smoking cessation, for example, strengthened regulations on cigarette sales and establishment of smoking prohibited areas. However, it is not easy for smokers who are addicted to nicotine contained in cigarettes to quit smoking overnight.

In this context, recently, electronic cigarettes that do not contain poisonous ingredients such as tar found in tobacco cigarettes and produce smoke by vaporizing a solution containing nicotine have been developed and are being sold. Electronic cigarettes may satisfy a smoker's urge to smoke while emitting less harmful smoke, thereby reducing damage to smokers and secondhand smokers, and are even used as a smoking cessation aid for smokers who want to quit smoking.

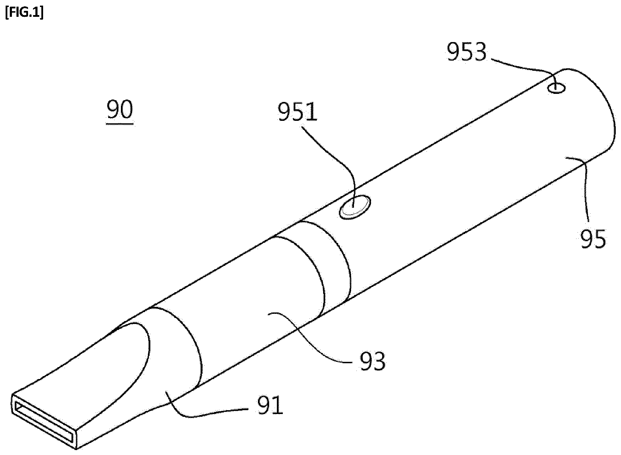

FIG. 1 is a view showing a conventional electronic cigarette 90 which is disclosed in Korean Patent Application Publication No. 10-2016-0095694 (August 12, 2016).

Referring to FIG. 1, the conventional electronic cigarette 90 includes: a mouthpiece part 91 having a shape that a user may bite into; a cartridge part 93 that stores liquid to be vaporized therein and delivers the vaporized liquid, or an aerosol (vapor) to the mouthpiece part 91; and a battery part 95 connected to the cartridge part 93 and supplies power necessary to vaporize the liquid.

As shown in FIG. 1, the battery part 95 of the conventional electronic cigarette 90 is provided with a button portion 951, so that when a user wants to use the electronic cigarette 90, he or she may press the button portion 951 to vaporize the liquid. In addition, a lamp portion 953 is provided in the battery part 95, allowing the user to check whether the electronic cigarette 90 is operating, an operating status of the electronic cigarette 90, etc.

However, as shown in FIG. 1, in the conventional electronic cigarette 90, since the mouthpiece part 91 remains exposed to the outside, there is a high possibility that the mouthpiece part 91 will be contaminated with foreign substances during use or transportation. In particular, the mouthpiece part 91 needs to be kept clean at all times because the mouthpiece part 91 is the part where a user inhales aerosol with his or her mouth, but the conventional electronic cigarette 90 does not have a separate means to protect the mouthpiece part 91, which is problematic.

Moreover, there is also a problem of liquid leaking through an opening of the mouthpiece part 91 exposed to the outside while a user carries the electronic cigarette 90 as the liquid used for vaporization is stored inside the cartridge part 93 of the liquid-type electronic cigarette, and the mouthpiece part 91 of the electronic cigarette 90 is always exposed to the outside.

Above all, in the conventional electronic cigarette 90, as shown in FIG. 1, since the button portion 951 protrudes on the outer surface of the battery part 95, when the electronic cigarette 90 is placed in a bag or pocket, the button portion 951 is pressed for various reasons and the electronic cigarette 90 operates regardless of the user's will.

This unwanted operation of the electronic cigarette 90 causes unnecessary power consumption and requires frequent charging of the electronic cigarette. In fact, when a user wants to use the electronic cigarette 90, there are cases where the charged power is consumed due to this unwanted operation and the battery is discharged.

In this regard, in the related industries, there is a demand for the development of new types of electronic cigarettes such that a cover is provided on an electronic cigarette to prevent contamination of the mouthpiece part 91 and leakage of liquid, and the problem where the button portion 951 protruding on the outside of the electronic cigarette being pressed unintentionally and consuming power is prevented.

(Patent Document 1) Korean Patent Application Publication No. 10-2016-0095694 (August 12, 2016)

### Disclosure

### Technical Problem

The present disclosure has been made to solve the above problems, and
an objective of the present disclosure is to provide an electronic cigarette in which, in a state where a cover part is coupled to a body part, thereby covering a cartridge part, an electric current is prevented from flowing toward the cartridge part even when a button formed on the electronic cigarette is pressed, and thus the problem where aerosol is generated as a result of the current being supplied toward the cartridge part by the button being pressed against the intention of a user is prevented, electrical discharge of the electronic cigarette as a result of unnecessary power consumption is prevented.

Another objective of the present disclosure is to provide an electronic cigarette in which, in a state where a cover part is separated from a body part, thereby exposing a cartridge part to the outside, an electric current flows toward the cartridge part when a user presses a button formed on the electronic cigarette, and thus aerosol may be generated.

Still another objective of the present disclosure is to provide an electronic cigarette in which a cartridge part located inside a cover part is automatically sterilized by allowing a sterilization part provided inside the cover part to operate when a user stops using the electronic cigarette and attaches the cover part to a body part.

Still another objective of the present disclosure is to provide an electronic cigarette in which a sterilization part operates only for a predetermined period of time from the time point when a cover part is coupled to a body part, preventing electrical energy stored in a battery part from being excessively used for sterilization work.

Still another objective of the present disclosure is to provide an electronic cigarette that allows a user to proceed with sterilization for a preset period of time by pressing a button on the electronic cigarette when a cover part is coupled to a body part and keeps covering a cartridge part even after an automatic sterilization process is completed.

Still another objective of the present disclosure is to provide an electronic cigarette in which coupling or decoupling of a cover part and a body part is confirmed by checking whether a first terminal portion and a second terminal portion are in contact by constructing the first terminal portion on one side of the body part that is in contact with the cover part, and constructing the second terminal portion on one side of the cover part opposing the first terminal portion.

Still another objective of the present disclosure is to provide an electronic cigarette in which, when a first terminal portion and a second terminal portion are in contact, the supply of electrical energy to a cartridge part is blocked, but the electrical energy is allowed to be supplied to a sterilization part formed in a cover part, whereas when the first terminal portion and the second terminal portion are not in contact, the supply of electrical energy to the sterilization part is blocked, but the electrical energy is allowed to be supplied to the cartridge part.

Still another objective of the present disclosure is to provide an electronic cigarette in which, by constructing a first magnet portion on one side of a body part that is in contact with a cover part, and constructing a second magnet portion on one side of the cover part opposing the first magnet portion, the cover part is easily coupled to the body part by magnetic attraction of the first magnet portion and the second magnet portion, and the cover part is separated from the body part only when a force greater than the attraction force of the first magnet portion and the second magnet portion is applied, so that unintentional separation of the cover part is prevented.

Still another objective of the present disclosure is to provide an electronic cigarette in which, by constructing a third magnet portion on one side of a body part that is in contact with a cartridge part, and constructing a fourth magnet portion on one side of the cartridge part opposing the third magnet portion, the cartridge part is easily coupled to the body part by magnetic attraction of the third magnet portion and the fourth magnet portion, and the cartridge part is separated from the body part only when a force greater than the attraction force of the third magnet portion and the fourth magnet portion is applied, so that unintentional separation of the cartridge part is prevented.

Still another objective of the present disclosure is to provide an electronic cigarette in which a housing part forming the outer surface of a body part is configured to protect the inside of the device, a protrusion portion extending downward is formed on a cover part, and on the upper surface of the housing part, a receiving portion is formed to be recessed in a shape complementary to the shape of the protrusion portion, so that the cover part and the body part, which are coupled by magnetic attraction, are guided by the protrusion portion and the receiving portion to ensure proper position coupling, and movement in the coupled state is prevented.

Still another objective of the present disclosure is to provide an electronic cigarette in which, by providing a seating portion inside a housing part of a body part and allowing a cartridge part to be accommodated on the seating portion, the cartridge part is stably fixed on the body part.

Still another objective of the present disclosure is to provide an electronic cigarette in which, by constructing a third terminal portion connected to a control part to be exposed on a seating portion where a cartridge part is accommodated, and constructing a fourth terminal portion on one side of the cartridge part opposite to the third terminal portion, the third terminal portion and the fourth terminal portion are brought into contact when the cartridge part is coupled to a body part, so that electrical energy is supplied from a battery part to the cartridge part under the control of the control part.

### Technical Solution

The present disclosure is implemented by an embodiment having the following configuration to achieve the above mentioned objectives.

According to an embodiment of the present disclosure, there is provided an electronic cigarette including: a body part that forms a body of the electronic cigarette; a cartridge part that is coupled to the body part and produces an aerosol; and a cover part that is detachably coupled to the body part and covers the cartridge part or exposes the cartridge part to the outside, wherein the body part may include: a battery part that supplies electrical energy; and a control part that determines a movement path of the electrical energy supplied from the battery part.

According to another embodiment of the present disclosure, the control part may block supply of electrical energy from the battery part to the cartridge part when the cover part is coupled to the body part.

According to still another embodiment of the present disclosure, the control part may allow the supply of electrical energy from the battery part to the cartridge part when the cover part is separated from the body part.

According to still another embodiment of the present disclosure, the control part may include: a cover open/close confirmation portion to check whether the cover part is open/closed.

According to still another embodiment of the present disclosure, the control part may include: a cartridge control portion that is connected to the cover open/close confirmation portion to block the supply of electrical energy to the cartridge part when the cover part is coupled to the body part and to supply electrical energy to the cartridge part when the cover part is separated from the body part.

According to still another embodiment of the present disclosure, the cartridge control portion may include: a cartridge power cut-off module that blocks the supply of electrical energy to the cartridge part when the cover part is confirmed to be coupled to the body part by the cover open/close confirmation portion; a heating signal confirmation module that checks whether a heating signal is being received when the cover part is confirmed to be separated from the body part by the cover open/close confirmation portion; and a cartridge power supply module that is connected to the heating signal confirmation module and supplies electrical energy to the cartridge part when it is confirmed that a heating signal has been received.

According to still another embodiment of the present disclosure, the cover part may further include: a sterilization part that emits light into an inner space of the cover part, and the control part may include: a sterilization control portion that is connected to the cover open/close confirmation portion and supplies or blocks electrical energy to the sterilization part when the cover part is coupled to the body part.

According to still another embodiment of the present disclosure, the sterilization control portion may include: a sterilization part power supply module that supplies electrical energy to the sterilization part when the cover part is coupled to the body part; a time check module that is connected to the sterilization part power supply module and checks a period of time during which electrical energy is supplied to the sterilization part; and a sterilization part power cut-off module that is connected to the time check module and blocks the electrical energy being supplied to the sterilization part when a preset time is exceeded.

According to still another embodiment of the present disclosure, the sterilization control portion may include: a sterilization signal confirmation module that is connected to the sterilization part power supply module and sends a signal to supply electrical energy to the sterilization part to the sterilization part power supply module when a sterilization signal is received after the supply of electrical energy to the sterilization part is blocked by the sterilization part power cut-off module.

According to still another embodiment of the present disclosure, the body part may include a housing part that forms an outer surface of the electronic cigarette, and the cover part may include a protrusion portion extending toward the housing part, wherein the housing part may include a receiving portion formed to be recessed in a shape complementary to a shape of the protrusion portion to accommodate the protrusion portion.

According to still another embodiment of the present disclosure, the housing part may include a seating portion recessed to form a seating space for accommodating the cartridge part.

According to still another embodiment of the present disclosure, the body part may include a first terminal portion provided on a surface thereof that is in contact with the cover part while the cover part may include a second terminal portion provided on a position opposite to the first terminal portion, and thus the first terminal portion and the second terminal portion may be in contact with each other when the cover part is coupled to the body part; the first terminal portion and the second terminal portion may not be in contact when the cover part is separated from the body part.

According to still another embodiment of the present disclosure, the body part may include a third terminal portion having a first side connected to the control part and a second side exposed while the cartridge part may include a fourth terminal portion that is contact-connected to the third terminal portion, and thus the third terminal portion and the fourth terminal portion may be in contact with each other when the cartridge part is coupled to the body part; the third terminal portion and the fourth terminal portion may not be in contact when the cartridge part is separated from the body part.

According to still another embodiment of the present disclosure, the body part may include a first magnet portion provided on a surface thereof that is in contact with the cover part while the cover part may include a second magnet portion provided on a position opposite to the first magnet portion, so that the body part and the cover part may be separably coupled by magnetic attraction of the first magnet portion and the second magnet portion.

According to still another embodiment of the present disclosure, the body part may include a third magnet portion provided on a surface thereof that is in contact with the cartridge part while the cartridge part may include a fourth magnet portion provided on a position opposite to the third magnet portion, so that the body part and the cartridge part may be separably coupled by magnetic attraction of the third magnet portion and the fourth magnet portion.

### Advantageous Effects

The present disclosure can achieve the following effects by the above-mentioned embodiments and the configuration, combination, and use relationship described below.

According to the present disclosure, by preventing an electric current from flowing toward a cartridge part even when a button formed on an electronic cigarette is pressed in a state where a cover part is coupled to a body part, thereby covering the cartridge part, it is possible to prevent the problem where aerosol is generated as a result of the current being supplied toward the cartridge part by the button being pressed against the intention of a user, and to prevent electrical discharge of the electronic cigarette as a result of unnecessary power consumption.

According to the present disclosure, in a state where a cover part is separated from a body part, thereby exposing a cartridge part to the outside, an electric current flows toward the cartridge part when a user presses a button formed on an electronic cigarette, and thus aerosol can be generated.

According to the present disclosure, a cartridge part located inside a cover part can automatically sterilized by allowing a sterilization part provided inside the cover part to operate when a user stops using an electronic cigarette and attaches the cover part to a body part.

According to the present disclosure, since a sterilization part operates only for a predetermined period of time from the time point when a cover part is coupled to a body part, it is possible to prevent electrical energy stored in a battery part from being excessively used for sterilization work.

According to the present disclosure, a user can proceed with sterilization for a preset period of time by pressing a button on an electronic cigarette when a cover part is coupled to a body part and keeps covering a cartridge part even after an automatic sterilization process is completed.

According to the present disclosure, coupling or decoupling of a cover part and a body part can be confirmed by checking whether a first terminal portion and a second terminal portion are in contact by constructing the first terminal portion on one side of the body part that is in contact with the cover part, and constructing the second terminal portion on one side of the cover part opposing the first terminal portion.

According to the present disclosure, when a first terminal portion and a second terminal portion are in contact, the supply of electrical energy to a cartridge part is blocked, but the electrical energy can be supplied to a sterilization part formed in a cover part, whereas when the first terminal portion and the second terminal portion are not in contact, the supply of electrical energy to the sterilization part is blocked, but the electrical energy can be supplied to the cartridge part.

According to the present disclosure, by constructing a first magnet portion on one side of a body part that is in contact with a cover part, and constructing a second magnet portion on one side of the cover part opposing the first magnet portion, the cover part can be easily coupled to the body part by magnetic attraction of the first magnet portion and the second magnet portion, and the cover part can be separated from the body part only when a force greater than the attraction force of the first magnet portion and the second magnet portion is applied, thereby preventing unintentional separation of the cover part.

According to the present disclosure, by constructing a third magnet portion on one side of a body part that is in contact with a cartridge part, and constructing a fourth magnet portion on one side of the cartridge part opposing the third magnet portion, the cartridge part can be easily coupled to the body part by magnetic attraction of the third magnet portion and the fourth magnet portion, and the cartridge part can be separated from the body part only when a force greater than the attraction force of the third magnet portion and the fourth magnet portion is applied, thereby preventing unintentional separation of the cartridge part.

According to the present disclosure, since a housing part forming the outer surface of a body part is configured to protect the inside of the device, a protrusion portion extending downward is formed on a cover part, and on the upper surface of the housing part, a receiving portion is formed to be recessed in a shape complementary to the shape of the protrusion portion, the cover part and the body part, which are coupled by magnetic attraction, can be guided by the protrusion portion and the receiving portion to ensure proper position coupling, and movement in the coupled state can be prevented.

According to the present disclosure, by providing a seating portion inside a housing part of a body part and allowing a cartridge part to be accommodated on the seating portion, the cartridge part can be stably fixed on the body part.

According to the present disclosure, by constructing a third terminal portion connected to a control part to be exposed on a seating portion where a cartridge part is accommodated, and constructing a fourth terminal portion on one side of the cartridge part opposite to the third terminal portion, the third terminal portion and the fourth terminal portion can be brought into contact when the cartridge part is coupled to a body part, and electrical energy can be supplied from a battery part to the cartridge part under the control of the control part.

### Description of Drawings

FIG. 1 is a view showing a conventional electronic cigarette;
FIG. 2 is a perspective view showing an electronic cigarette according to an embodiment of the present disclosure;
FIG. 3 is an exploded perspective view of FIG. 2;
FIG. 4 is a view showing a body part;
FIG. 5 is a view showing a control part;
FIG. 6 is a flowchart showing the control process of the control part;
FIG. 7 is a view showing a cartridge part;
FIG. 8 is view showing a cover part;
FIG. 9 is a state of use of the present disclosure showing that sterilization of the cartridge part is performed by means of a sterilization part when the cover part is coupled to the body part; and
FIG. 10 is a state of use of the present disclosure showing that when the cover part is separated from the body part, electrical energy is supplied to the cartridge part and vaporization occurs.

### Best Mode

Hereinafter, preferred embodiments of an electronic cigarette according to the present disclosure will be described in detail with reference to the attached drawings. In the following description of the present disclosure, if a detailed description of a known function or configuration is judged to unnecessarily obscure the gist of the present disclosure, the detailed description will be omitted. Unless otherwise specified, all terms in this specification have the same general meaning as understood by a person skilled in the art to which the present disclosure pertains, and if there is a conflict with the meaning of the terms used in this specification, the definitions used in this specification shall apply.

According to an electronic cigarette 1 of the present disclosure, in a state where a cover part 50 is coupled to a body part 10, thereby covering a cartridge part 30, an electric current is prevented from flowing toward the cartridge part 30 even when a button formed on the electronic cigarette 1 is pressed, and thus the problem where aerosol is generated as a result of the current being supplied toward the cartridge part 30 by the button being pressed against the intention of a user is prevented, electrical discharge of the electronic cigarette as a result of unnecessary power consumption is prevented. On the other hand, in a state where the cover part 50 is separated from the body part 10, thereby exposing the cartridge part 30 to the outside, an electric current flows toward the cartridge part 30 when a user presses a button formed on the electronic cigarette, and thus aerosol may be generated.

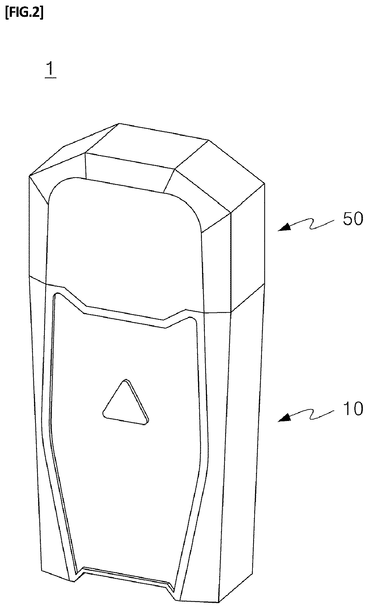

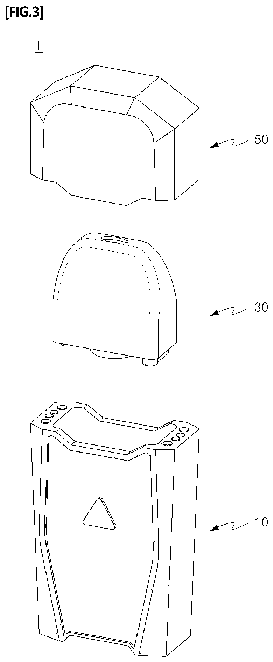

FIG. 2 is a perspective view showing the electronic cigarette 1 according to an embodiment of the present disclosure, and FIG. 3 is an exploded perspective view of FIG. 2. Referring to FIGS. 2 and 3, the electronic cigarette 1 includes the body part 10, the cartridge part 30, and the cover part 50.

The body part 10 is a component that forms the body of the electronic cigarette 1. As shown in FIGS. 2 and 3, the cartridge part 30, which will be described later, is detachably coupled to the upper side of the body part 10, and the cover part 50, which will be described later, may also be detachably coupled to the body part 10. Due to this, the body part 10 may be repeatedly reused, and only the cartridge part 30, which will be described later and which is coupled to the body part 10, may be replaced. When using the electronic cigarette 1, the cover part 50 coupled to the body part 10 may be separated, and upon completion of use, the cover part 50 may be coupled to the body part 10 again.

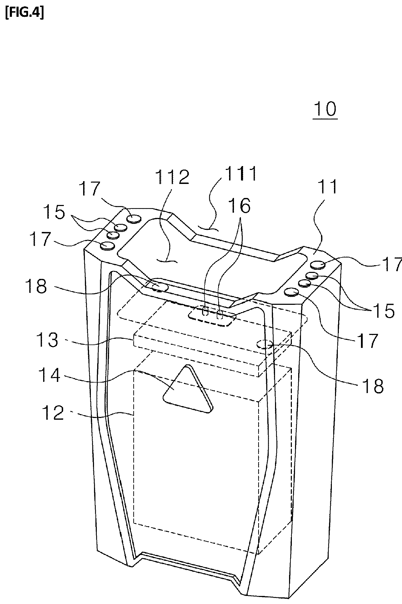

FIG. 4 is a view showing the body part 10. Referring to FIG. 4, the body part 10 includes a housing part 11, a battery part 12, a control part 13, an input part 14, a first terminal portion 15, a first magnet portion 17, and a third magnet portion 18.

The housing part 11 forms the outer surface of the body of the electronic cigarette and protects the battery part 12, the control part 13, etc. located inside the housing part 11. As shown in FIG. 4, the housing part 11 is shown in the shape of a roughly rectangular parallelepiped, but the shape of the housing part 11 is not necessarily limited to this shape, and as long as the housing part 11 performs the above-described functions, the housing part 11 may be configured in any number of different shapes. As shown in FIG. 4, the lower side of the housing portion 11 may be provided with a portion recessed upward in the center, and a charging terminal for charging the battery part 12, which will be described later, may be located in this recessed portion.

The housing part 11 includes a receiving portion 111 and a seating portion 112.

The receiving portion 111 refers to a portion recessed on the upper part of the housing part 11 in a shape complementary to the shape of a protrusion portion 54 to accommodate the protrusion portion 54 of the cover part 50, which will be described later. The protrusion portion 54 extending downward is formed on the cover part 50, and the receiving portion 111 is formed on the upper surface of the housing part 11, so that the cover part 50 and the body part 10, which are coupled by magnetic attraction, are preferably guided by the protrusion portion 54 and the receiving portion 111 to ensure proper position coupling, and movement in the coupled state is prevented.

The seating portion 112 refers to a configuration that is recessed into a box shape as shown in FIG. 4 to form a seating space for accommodating the cartridge part 30, which will be described later. The lower part of the cartridge part 30, which will be described later, is accommodated on the seating portion 112, so that the cartridge part 30 may be stably fixed on the body part 10. To this end, it is preferable that the shape of the seating portion 112 is complementary to the shape of the lower part of the cartridge part 30.

The battery part 12 is configured to supply electrical energy to parts that require electrical energy for the operation of the electronic cigarette 1. As shown in FIG. 4, the battery part 12 is located inside the housing part 11 and stores electrical energy, and is configured to transmit the stored electrical energy to the cartridge part 30 or a sterilization part 52, which will be described later. Although not shown, it may be desirable that a connection terminal is provided on the lower side of the housing part 11 so that electrical energy may be supplied from the outside and charged to the battery part 12, and for the battery part 12 to be connected to the connection terminal.

The control part 13 refers to a component that determines the movement path of the electrical energy supplied from the battery part 12. Preferably, the control part 13 may be configured to block the supply of electrical energy from the battery part 12 to the cartridge part 30 when the cover part 50, which will be described later, is coupled to the body part 10, and to allow electrical energy to be supplied from the battery part 12 to the cartridge part 30 when the cover part 50, which will be described later, is separated from the body part 10. The control part 13 may have the form of a circuit board.

To be specific, the control part 13 blocks the supply of electrical energy to the cartridge part 30 when the first terminal portion 15 and a second terminal portion 51 are contact-connected, and enables supply of electrical energy to the cartridge part 30 when the first terminal portion 15 and the second terminal portion 51, which will be described later, are separated.

In addition, preferably, the control part 13 may be configured to block the supply of electrical energy to the sterilization part 52, which will be described later, from the battery part 12 when a predetermined period of time has elapsed based on the time when the first terminal portion 15 and the second terminal portion 51, which will be described later, are contacted.

The control part 13 includes a cover open/close confirmation portion 131, a cartridge control portion 132, and a sterilization control portion 133.

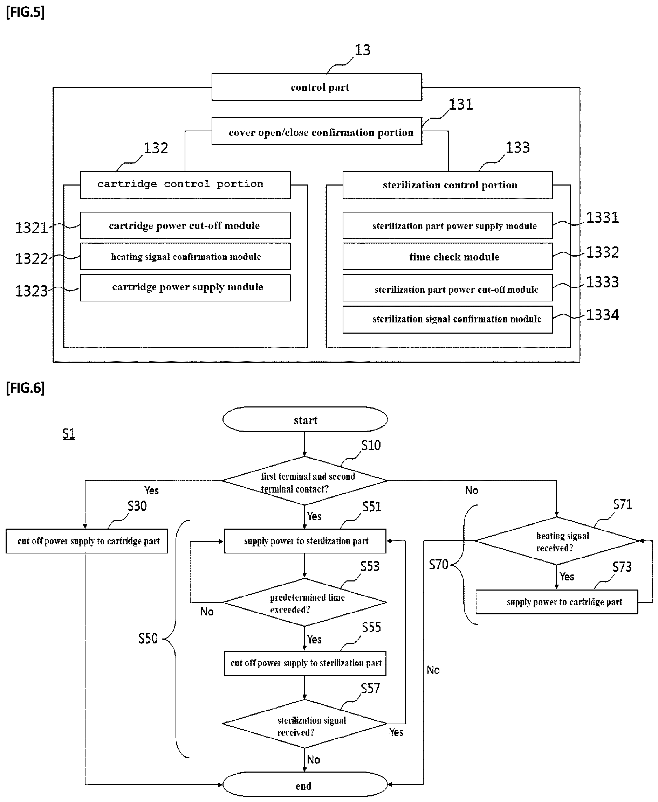

The cover open/close confirmation portion 131 is configured to check whether the cover part 50, which will be described later, is open/closed. Preferably, the cover open/close confirmation portion 131 may check whether the cover part 50 is open or closed from the body part 10 by checking whether the first terminal portion 15 and the second terminal portion 51, which will be described later, are in contact. As shown in FIG. 5, the cover open/close confirmation portion 131 may be connected to the cartridge control portion 132 and the sterilization control portion 133. FIG. 6 shows a control process S1 of the control part 13. Step S10 is performed to check, by the cover open/close confirmation portion 131, whether the first terminal portion 15 and the second terminal portion 51 are in contact. When it is confirmed that the first terminal portion 15 and the second terminal portion 51 are in contact, step S30 of blocking the supply of electrical energy to the cartridge part 30 is performed, and preferably, step S50 of supplying electrical energy to the sterilization part 52 may proceed at the same time. When it is confirmed by the cover open/close confirmation portion 131 that the first terminal portion 15 and the second terminal portion 51 are not in contact, step S70 of supplying electrical energy to the cartridge part 30 may proceed.

The cartridge control portion 132 is connected to the cover open/close confirmation portion 131 to block the supply of electrical energy to the cartridge part 30 when the cover part 50 is coupled to the body part 10 and to supply electrical energy to the cartridge part 30 when the cover part 50 is separated from the body part 10. More preferably, the cartridge control portion 132 may be viewed as a configuration that blocks the supply of electrical energy to the cartridge part 30 when the first terminal portion 15 and the second terminal portion 51 come into contact, and supplies electrical energy to the cartridge part 30 when the first terminal portion 15 and the second terminal portion 51 are not in contact.

Referring to FIG. 5, the cartridge control portion 132 includes a cartridge power cut-off module 1321, a heating signal confirmation module 1322, and a cartridge power supply module 1323.

The cartridge power cut-off module 1321 is configured to block the supply of electrical energy to the cartridge part 30 when it is confirmed by the cover open/close confirmation portion 131 that the cover part 50 is coupled to the body part 10. That is, the cartridge power cut-off module 1321 is activated and blocks the supply of electrical energy to the cartridge part 30 when it is confirmed by the cover open/close confirmation portion 131 that first terminal portion 15 and the second terminal portion 51 are in contact. Accordingly, in a state where the cover part 50, which will be described later, is coupled to the body part 10 because a user of the electronic cigarette 1 has no intention of using the electronic cigarette 1, electrical energy is not supplied to the cartridge part 30 even if a user presses the input part 14, which will be described later.

The heating signal confirmation module 1322 is configured to check whether a heating signal is being received when it is confirmed by the cover open/close confirmation portion 131 that the cover part 50 is separated from the body part 10. The heating signal may be generated by the input part 14, which will be described later. Preferably, the heating signal confirmation module 1322 is activated when it is confirmed by the cover open/close confirmation portion 131 that the first terminal portion 15 and the second terminal portion 51 are not in contact. As shown in FIGS. 5 and 6, the heating signal confirmation module 1322 may be viewed as a configuration that checks (S71) whether the heating signal generated by the input part 14, which will be described later, is being received.

The cartridge power supply module 1323 is connected to the heating signal confirmation module 1322 and supplies (S73) electrical energy to the cartridge part 30 when it is confirmed by the heating signal confirmation module 1322 that a heating signal has been received. When it is confirmed by the heating signal confirmation module 1322 that the heating signal is continuously received, the cartridge power supply module 1323 continues to supply electrical energy to the cartridge part 30, and thus the supplied electrical energy heats a coil, vaporizing liquid around the coil and generating smoke (aerosol).

The sterilization control portion 133 is connected to the cover open/close confirmation portion 131 and supplies or blocks electrical energy to the sterilization part 52 when the cover part 50 is coupled to the body part 10. Preferably, the sterilization control portion 133 may be viewed as a configuration that is activated (S50) when the first terminal portion 15 and the second terminal portion 51 come into contact. Referring to FIG. 5, the sterilization control portion 133 includes a sterilization part power supply module 1331, a time check module 1332, a sterilization part power cut-off module 1333, and a sterilization signal confirmation module 1334.

The sterilization part power supply module 1331 supplies electrical energy to the sterilization part 52 when the cover part 50 is coupled to the body part 10. The sterilization part power supply module 1331 refers to a configuration that is activated and supplies (S51) electrical energy to the sterilization part 52, which will be described later, when it is confirmed by the cover open/close confirmation portion 131 that the first terminal portion 15 and the second terminal portion 51 are not in contact. The sterilization part power supply module 1331 allows the sterilization part 52 to emit light to proceed with the sterilization work for the cartridge part 30 located inside the cover part 50.

The time check module 1332 is connected to the sterilization part power supply module 1331 and checks (S53) a period of time for which electrical energy is supplied to the sterilization part 52. The period of time may be set in advance, and the set time may be changed at any time. For example, if the time is set to 50 seconds, the time check module 1332 counts the time from when the first terminal portion 15 and the second terminal portion 51 come into contact until 50 seconds have passed, and when the set time arrives, notifies the sterilization part power cut-off module 1333, which will be described later, that the set time has elapsed.

The sterilization part power cut-off module 1333 is connected to the time check module 1332 and cuts off (S55) the power supply to the sterilization part 52 when the preset time has elapsed. Accordingly, the sterilization part power cut-off module 1333 allows the sterilization part 52 to operate only for a predetermined period of time from the time the cover part 50 covers the body part, thereby preventing excessive electrical energy from being used for sterilizing work.

The sterilization signal confirmation module 1334 is connected to the sterilization part power supply module 1331 and sends (S57) a signal to the sterilization part power supply module 1331 to supply electrical energy to the sterilization part 52 when a sterilization signal generated by the input part 14 is received after the supply of electrical energy to the sterilization part 52 is blocked by the sterilization part power cut-off module 1333. When a signal to supply electrical energy to the sterilization part 52 is detected by the sterilization signal confirmation module 1334, the sterilization signal confirmation module 1334 sends a sterilization signal to the sterilization part power supply module 1331 to enable sterilization by the sterilization part 52, and thus a user may perform manual sterilization whenever desired in addition to the automatic sterilization that occurs automatically when the cover part 50 covers the body part.

The input part 14 is connected to the control part 13 and is exposed to the outside of the body part 10. Preferably, as shown in FIG. 4, the input part 14 may be viewed as a button formed on the body part 10. The input part 14 is configured to generate a sterilization signal to supply electrical energy from the battery part 12 to the sterilization part 52 which will be described later and send the generated sterilization signal to the control part 13 when the first terminal portion 15 and the second terminal portion 51, which will be described later, are connected by contact, and generate a heating signal to supply electrical energy from the battery part 12 to the cartridge part 30 which will be described later and send the generated heating signal to the control part 13 when the first terminal portion 15 and the second terminal portion 51, which will be described later, are separated. In addition, a signal to turn the device on or off may be generated by the input part 14.

The input part 14 may be configured to generate an input signal when pressed with physical force, or may be configured to generate an input signal when touched using a touch method. In addition, the input part 14 may include a light emitting means and be configured to emit light of various colors representing the state of the electronic cigarette 1. For example, the input part 14 may be configured to emit red light when the cartridge part 30 accommodated in the seating portion 112 is separated, or blue light when the cartridge part 30 is seated and coupled to the seating portion 112, or to emit a specific light when a user presses or touches the input part 14.

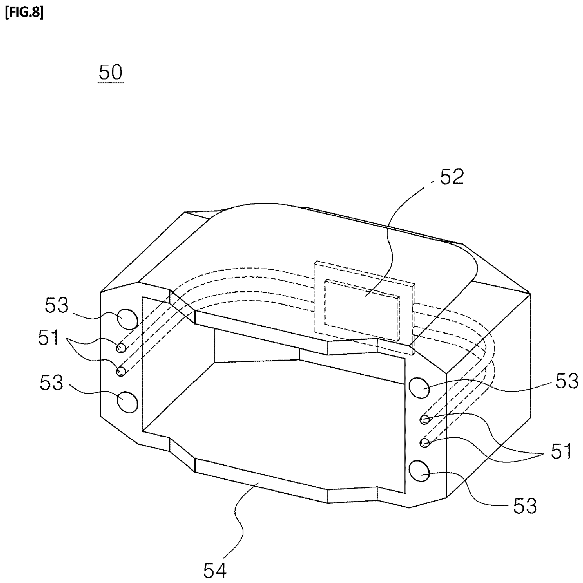

The first terminal portion 15 refers to a configuration provided on one surface of the body part 10 that is in contact with the cover part 50, which will be described later. Preferably, as shown in FIG. 4, the first terminal portion 15 is provided on the upper surface of the body part 10, and as shown in FIG. 8, the second terminal portion 51 is provided on the lower surface of the cover part 50 which will be described later, so that when the cover part 50 which will be described later is coupled to the body part 10, the first terminal portion 15 and the second terminal portion 51 may come into contact. Accordingly, coupling or decoupling of the cover part 50 and the body part 10 may be confirmed by checking whether the first terminal portion 15 and the second terminal portion 51 are in contact. Preferably, the first terminal portion 15 may be formed at the same level as the surroundings thereof without protruding, and a plurality of first terminal portions 15 may be formed on one side and on the other side of the upper surface of the body part 10, as shown in FIG. 4.

A third terminal portion 16 refers to a configuration that penetrates the housing part 11 and is exposed to the seating space. The third terminal portion 16 has one side connected to the control part 13 and the other side formed to protrude above the seating portion 112. Thus, when the cartridge part 30, which will be described later, is seated on the seating portion 112 of the housing part 11 by magnetic force, a fourth terminal portion 32 coupled to the lower side of the cartridge part 30 may contact the third terminal portion 16. Since the third terminal portion 16 is configured to protrude above the seating portion 112 by an elastic means, when the third terminal portion 16 is pressed by an external force, the protruding height is lowered by the external force, and when the external force is removed, the original protruding height is restored. Due to this, close contact between the fourth terminal portion 32, which will be described later, and the third terminal portion 16 may be achieved.

The first magnet portion 17 is provided on one surface of the body part 10 that is in contact with the cover part 50, which will be described later, and may form a magnetic attraction with a second magnet portion 53 provided on the lower surface of the cover part 50, which will be described later. By magnetic attraction of the first magnet portion 17 and the second magnet portion 53, the cover part 50 may be easily coupled to the body part 10 and the cover part 50 may be separated from the body part 10 only when a force greater than the attraction force of the first magnet portion 17 and the second magnet portion 53 is applied, so that unintentional separation of the cover part 50 is prevented. Preferably, as shown in FIG. 4, a plurality of first magnet portions 17 may be formed on one side and on the other side of the upper surface of the body part 10 such that the plurality of first terminal portions 15 formed on one side and on the other side of the upper surface of the body part 10 are located between the paired first magnet portions 17.

The third magnet portion 18 is provided on one surface of the body part 10 that is in contact with the cartridge part 30, which will be described later. The cartridge part 30 may be detachably coupled to the body part 10 by magnetic attraction between the third magnet portion and a fourth magnet portion 31 provided on the lower surface of the cartridge part 30, which will be described later. In the present disclosure, as the body part 10 and the cartridge part 30 are coupled to each other by such magnetic force, it is easy to couple or separate the cartridge part 30 from the body part 10, and the user's satisfaction with using the electronic cigarette 1 increases. Furthermore, since the cartridge part 30 is separated from the body part 10 only when a force greater than the attraction force of the third magnet portion 18 and the fourth magnet portion 31 is applied, unintentional separation of the cartridge part 30 may be prevented. As shown in FIG. 4, a plurality of third magnet portions 18 may be provided on the lower surface of the seating portion 112. More preferably, one third magnet portion 18 and another third magnet portion 18 may be located near opposite vertices respectively in the diagonal direction on the lower surface of the square seating portion 112.

The cartridge part 30 is a configuration that is coupled to the upper side of the body part 10 and produces aerosol (vapor), and is preferably viewed as being detachably coupled to the body part 10. The upper side of the cartridge part 30 becomes the part where a user bites with his/her mouth, and may be configured in a shape that is easy to bite with the mouth. The lower side of the cartridge part 30 may be configured in a shape complementary to the shape of the seating space formed by the seating portion 112. Preferably, a liquid solution is stored inside the cartridge part 30, and the liquid solution seeps into a liquid suction means wound around the coil. The electrical energy transmitted through the fourth terminal portion 32, which will be described later, heats the coil and applies heat to the liquid suction means into which the liquid solution has penetrated, so that the liquid solution is vaporized. The vaporized solution, that is, aerosol (vapor) moves upward along a pipe provided inside the cartridge part 30, is discharged to the outside through a hole formed on the upper side of the cartridge part 30, and may flow into the user's mouth.

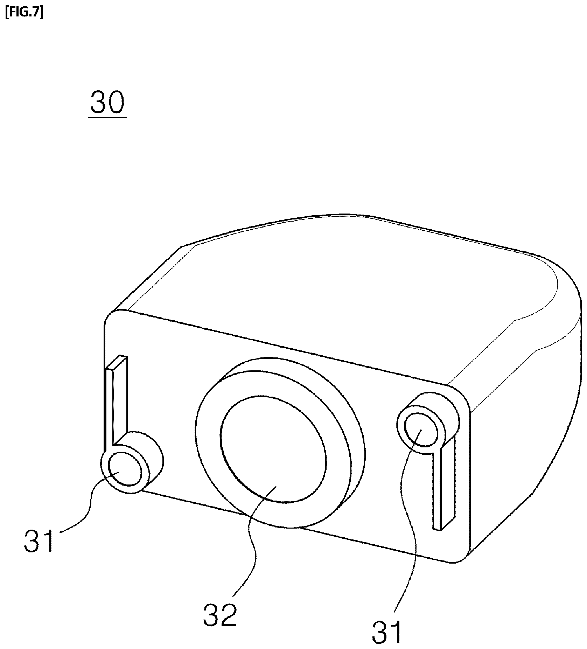

FIG. 7 is a view showing the cartridge part 30. Referring to FIG. 7, the cartridge part 30 includes the fourth magnet portion 31 and the fourth terminal portion 32.

The fourth magnet portion 31 is provided at a position opposite to the third magnet portion 18. The body part 10 and the cartridge part 30 may be separably coupled by the magnetic attraction of the third magnet portion 18 and the fourth magnet portion 31. As previously described, one third magnet portion 18 and another third magnet portion 18 may be provided near opposite vertices respectively in the diagonal direction on the lower surface of the square seating portion 112 as shown in FIG. 4, and thus one fourth magnet portion 31 and another fourth magnet portion 31 may be provided near opposite vertices respectively in the diagonal direction on the lower surface of the square cartridge part 30 as shown in FIG. 7. The fourth terminal portion 32, which will be described later, is coupled to the lower surface of the cartridge part 30, so that the lower surface of the cartridge part 30 is separated from the lower surface of the seating portion 112 by the height of the fourth terminal portion 32. In order to compensate for this, the fourth magnet portion 31 is preferably located at the end of an extension portion extending downward as shown in FIG. 3, so that when the third magnet portion 18 and the fourth magnet portion 31 are combined, the third terminal portion 16 and the fourth terminal portion 32 come into contact with each other.

The fourth terminal portion 32 is configured to be contact-connected to the third terminal portion 16 of the control part 13. By constructing the third terminal portion 16 connected to the control part 13 to be exposed on the seating portion 112 where the cartridge part 30 is accommodated, and constructing the fourth terminal portion 32 on one side of the cartridge part 30 opposite to the third terminal portion 16, the third terminal portion 16 and the fourth terminal portion 32 are brought into contact when the cartridge part 30 is coupled to the body part 10, so that electrical energy is supplied from the battery part 12 to the cartridge part 30 under the control of the control part 13. Referring to FIG. 7, the fourth terminal portion 32 is shown in a cylindrical shape, but the shape of the fourth terminal portion 32 is not limited to this shape, and may have various shapes.

The cover part 50 is detachably coupled to the body part 10 and covers the cartridge part 30 or exposes the cartridge part 30 to the outside. The cover part 50 may be viewed as a lid that covers and protects the cartridge part 30. As shown in FIG. 3, the cover part 50 is shown as being completely separated from the body part 10, but the present disclosure is not limited thereto. By connecting the cover part 50 and the body part 10 with a hinge, etc., the cartridge part 30 coupled to the body part 10 may be covered or exposed by the cover part 50 rotating around the hinge. To this end, an empty space may be formed inside the cover part 50, and this inner space may be configured in a shape and size to accommodate the cartridge part 30, while minimizing unnecessary separation space. In addition, to prevent the device from becoming bulky or heavy, the inner space may be configured to have a shape substantially similar to the outer shape of the cartridge part 30.

FIG. 8 is view showing the cover part 50. Referring to FIG. 8, the cover part 50 includes the second terminal portion 51, the sterilization part 52, the second magnet portion 53, and the protrusion portion 54.

The second terminal portion 51 is provided at a position opposite to the first terminal portion 15, and is formed so that the first terminal portion 15 and the second terminal portion 51 come into contact when the cover part 50 is coupled to the body part 10. When first terminal portion 15 and the second terminal portion 51 are in contact, the supply of electrical energy to the cartridge part 30 is blocked, but the electrical energy is allowed to be supplied to the sterilization part 52 formed in the cover part 50, whereas when the first terminal portion 15 and the second terminal portion 51 are not in contact, the supply of electrical energy to the sterilization part 52 is blocked, but the electrical energy is allowed to be supplied to the cartridge part 30. As shown in FIG. 8, a plurality of second terminal portions 51 may be formed on one side and the other side of the lower surface of the cover part.

As shown in FIG. 8, the second terminal portion 51 is configured to protrude downward from the lower surface of the cover part 50 by an elastic means (not shown), etc., and thus when the second terminal portion 51 is pressed by an external force, the protruding height is lowered by the external force, and when the external force is removed, the original protruding height is restored. Due to this, the first terminal portion 15 and the second terminal portion 51 may be brought into close contact.

One side of the second terminal portion 51 may be connected to the first terminal portion 15, and the other side of the second terminal portion 51 is configured to be connected to the sterilization part 52, which will be described later, by an electric wire. Thus, when the cover part 50 is couple to the body part 10, an electric current may flow to the sterilization part 52 through the first terminal portion 15, the second terminal portion 51, and the electric wire.

The sterilization part 52 is connected to the first terminal portion 15 and emits light into the inner space of the cover part 50. The sterilization part 52 is formed to receive electric energy from the battery part 12 and emit light when the first terminal portion 15 and the second terminal portion 51 are contacted. When a user stops using the electronic cigarette 1 and couples the cover part 50 to the body part 10, the sterilization part 52 formed inside the cover part 50 is activated and the cartridge part 30 located inside the cover part 50 is automatically sterilized.

Preferably, the operation of the sterilization part 52 may be performed only for a predetermined period of time from the time the cover part 50 is coupled to the body part 10. As a result, it is possible to prevent the electric energy charged in the battery part 12 from being excessively used for sterilization work. More preferably, the sterilization part 52 may be configured such that a user may proceed with sterilization for a preset period of time by pressing the input part 14 formed on the electronic cigarette 1 when the cover part 50 is coupled to the body part 10 and keeps covering the cartridge part 30 even after an automatic sterilization process is completed.

The second magnet portion 53 is provided at a position opposite to the first magnet portion 17. The body part 10 and the cover part 50 are separably coupled by magnetic attraction of the first magnet portion 17 and the second magnet portion 53. As shown in FIG. 8, a plurality of second magnet portions 53 may be provided on one side and the other side of the lower surface of the cover part so as to be located outside the second terminal portion 51 which is formed in plural numbers on one side and the other side of the lower surface of the cover part.

The protrusion portion 54 is configured to extend toward the housing part 11, and to have a shape complementary to the receiving portion 111 of the housing part 11. Thus, when the cover part 50 is coupled to the body part 10, the protrusion portion 54 may be configured to engage in male and female coupling with the receiving portion 111. As the protrusion portion 54 is received in the receiving portion 111, the problem of the cover part 50 being separated from the body part 10 due to an external force perpendicular to the direction of attraction of the first magnet portion 17 and the second magnet portion 53 may be prevented.

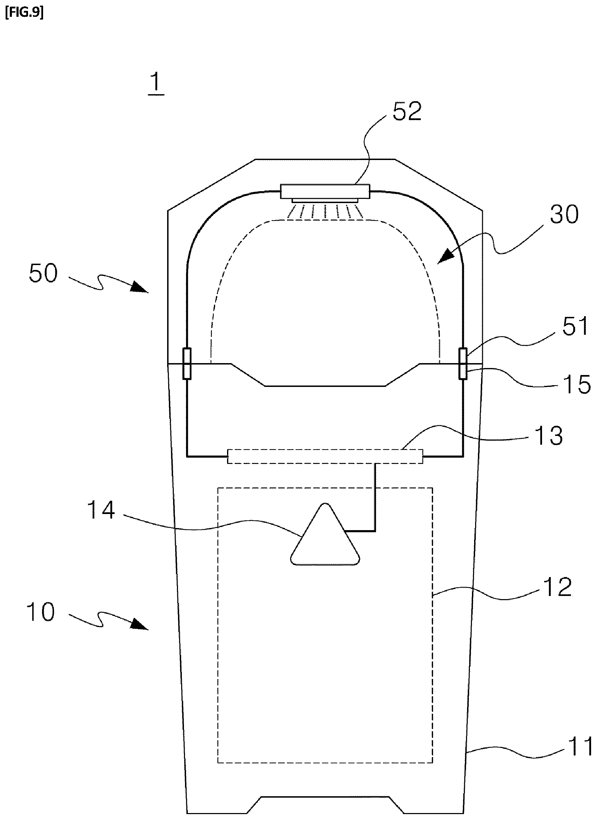

FIG. 9 is a state of use of the present disclosure showing that sterilization of the cartridge part 30 is performed by means of the sterilization part 52 when the cover part 50 is coupled to the body part 10. Referring to FIG. 9, since the supply of electrical energy to the cartridge part 30 is blocked by the control part 13 when the cover part 50 is coupled to the body part 10, electricity is not supplied to the cartridge part 30 even if a user presses the input part 14, and electrical energy may be supplied to the sterilization part 52 by contact between the first terminal portion 15 and the second terminal portion 51.

When the separated cover part 50 is coupled to the body part 10, electric energy is supplied to the sterilization part 52 for a predetermined period of time from that point, and the cartridge part 30 is sterilized by the sterilization part 52. For example, if the sterilization time is set to 50 seconds, sterilization is performed for 50 seconds from the time the cover part 50 is coupled to the body part 10, and after 50 seconds, light irradiation by the sterilization part 52 ends. Afterwards, when the cover part 50 is separated and reattached to the body part 10, sterilization is performed for 50 seconds from the time of reattachment.

If the cover part 50 continues to be coupled to the body part 10 even after automatic sterilization is completed, no further automatic sterilization operation occurs. However, at this time, a user may manually instruct the sterilization operation through the input part 14, and in this case, the sterilization operation may occur again for a preset time, for example, 50 seconds.

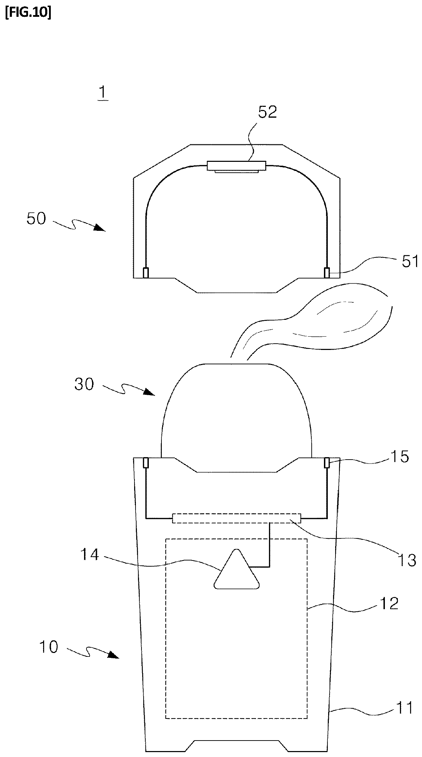

FIG. 10 is a state of use of the present disclosure showing that when the cover part 50 is separated from the body part 10, electrical energy is supplied to the cartridge part 30 and vaporization occurs. Referring to FIG. 10, when the cover part 50 is separated from the body part 10, the blockage of electric energy supply to the cartridge part 30 by the control part 13 is released. In this state, a user may instruct power supply to the cartridge part 30 through the input part 14, and accordingly, electric energy is supplied to the cartridge part 30, and the liquid solution is vaporized to produce aerosol so that the user may inhale the produced aerosol. In this case, because the first terminal portion 15 and the second terminal portion 51 are separated from each other, even though the sterilization process was in progress while the first terminal portion 15 and the second terminal portion 51 were in contact with each other, the supply of electrical energy to the sterilization part 52 is cut off by the separation of the cover part 50, thereby causing the sterilization work to stop.

The above detailed description is illustrative of the present disclosure. In addition, the foregoing describes preferred embodiments of the present disclosure, and the present disclosure may be used in various other combinations, modifications, and environments. That is, the present disclosure may be changed or modified within the scope of the inventive concept disclosed herein, the scope equivalent to the written disclosure, and/or the skill or knowledge in the art. The written embodiments illustrate the best state for implementing the technical idea of the present disclosure, and various changes required for specific application fields and uses of the present disclosure are also possible. Therefore, the above detailed description of the invention is not intended to limit the invention to the disclosed embodiments. Furthermore, the appended claims should be construed to include other embodiments as well.

## Claims

1. An electronic cigarette, comprising:
a body part that forms a body of the electronic cigarette;
a cartridge part that is coupled to the body part and produces an aerosol; and
a cover part that is detachably coupled to the body part and covers the cartridge part or exposes the cartridge part to the outside,
wherein the body part comprises:
a battery part that supplies electrical energy; and
a control part that determines a movement path of the electrical energy supplied from the battery part.

2. The electronic cigarette of claim 1, wherein the control part blocks supply of electrical energy from the battery part to the cartridge part when the cover part is coupled to the body part.

3. The electronic cigarette of claim 2, wherein the control part allows the supply of electrical energy from the battery part to the cartridge part when the cover part is separated from the body part.

4. The electronic cigarette of claim 3, wherein the control part comprises:
a cover open/close confirmation portion to check whether the cover part is open/closed.

5. The electronic cigarette of claim 4, wherein the control part comprises:
a cartridge control portion that is connected to the cover open/close confirmation portion to block the supply of electrical energy to the cartridge part when the cover part is coupled to the body part and to supply electrical energy to the cartridge part when the cover part is separated from the body part.

6. The electronic cigarette of claim 5, wherein the cartridge control portion comprises:
a cartridge power cut-off module that blocks the supply of electrical energy to the cartridge part when the cover part is confirmed to be coupled to the body part by the cover open/close confirmation portion;
a heating signal confirmation module that checks whether a heating signal is being received when the cover part is confirmed to be separated from the body part by the cover open/close confirmation portion; and
a cartridge power supply module that is connected to the heating signal confirmation module and supplies electrical energy to the cartridge part when it is confirmed that a heating signal has been received.

7. The electronic cigarette of claim 4, wherein the cover part further comprises:
a sterilization part that emits light into an inner space of the cover part, and
the control part comprises:
a sterilization control portion that is connected to the cover open/close confirmation portion and supplies or blocks electrical energy to the sterilization part when the cover part is coupled to the body part.

8. The electronic cigarette of claim 7, wherein the sterilization control portion comprises:
a sterilization part power supply module that supplies electrical energy to the sterilization part when the cover part is coupled to the body part;
a time check module that is connected to the sterilization part power supply module and checks a period of time during which electrical energy is supplied to the sterilization part; and
a sterilization part power cut-off module that is connected to the time check module and blocks the electrical energy being supplied to the sterilization part when a preset time is exceeded.

9. The electronic cigarette of claim 8, wherein the sterilization control portion comprises:
a sterilization signal confirmation module that is connected to the sterilization part power supply module and sends a signal to supply electrical energy to the sterilization part to the sterilization part power supply module when a sterilization signal is received after the supply of electrical energy to the sterilization part is blocked by the sterilization part power cut-off module.

10. The electronic cigarette of claim 1, wherein the body part comprises a housing part that forms an outer surface of the electronic cigarette, and the cover part comprises a protrusion portion extending toward the housing part,
wherein the housing part comprises a receiving portion formed to be recessed in a shape complementary to a shape of the protrusion portion to accommodate the protrusion portion.

11. The electronic cigarette of claim 10, wherein the housing part comprises a seating portion recessed to form a seating space for accommodating the cartridge part.

12. The electronic cigarette of claim 1, wherein the body part comprises a first terminal portion provided on a surface thereof that is in contact with the cover part while the cover part comprises a second terminal portion provided on a position opposite to the first terminal portion, and thus the first terminal portion and the second terminal portion are in contact with each other when the cover part is coupled to the body part; the first terminal portion and the second terminal portion are not in contact when the cover part is separated from the body part.

13. The electronic cigarette of claim 1, wherein the body part comprises a third terminal portion having a first side connected to the control part and a second side exposed while the cartridge part comprises a fourth terminal portion that is contact-connected to the third terminal portion, and thus the third terminal portion and the fourth terminal portion are in contact with each other when the cartridge part is coupled to the body part; the third terminal portion and the fourth terminal portion are not in contact when the cartridge part is separated from the body part.

14. The electronic cigarette of claim 1, wherein the body part comprises a first magnet portion provided on a surface thereof that is in contact with the cover part while the cover part comprises a second magnet portion provided on a position opposite to the first magnet portion, so that the body part and the cover part are separably coupled by magnetic attraction of the first magnet portion and the second magnet portion.

15. The electronic cigarette of claim 1, wherein the body part comprises a third magnet portion provided on a surface thereof that is in contact with the cartridge part while the cartridge part comprises a fourth magnet portion provided on a position opposite to the third magnet portion, so that the body part and the cartridge part are separably coupled by magnetic attraction of the third magnet portion and the fourth magnet portion.
